# EUROPEAN PATENT SPECIFICATION

(11) **EP 2 438 868 B1**
(45) Date of publication and mention of the grant of the patent: **08.10.2014**
(21) Application number: 09845374.9
(22) Date of filing: 05.06.2009
(51) Int. Cl.: A61B 17/56, A61F 2/46, A61M 25/00, A61B 17/88

(54) **VERTEBRA FILLER**
WIRBELFÜLLSTOFF
AGENT DE REMPLISSAGE DE VERTÈBRES

(43) Date of publication of application: 11.04.2012
(73) Proprietor: Shao, Weixing, Shandong 250014 (CN)
(72) Inventor: Shao, Weixing, Shandong 250014 (CN)
(74) Representative: Sun, Yiming
(86) International application number: PCT/CN2009/000626
(87) International publication number: WO 2010/139093

(56) References cited:
- CN-A- 1 371 752
- CN-A- 1 568 162
- CN-A- 1 662 193
- CN-A- 1 883 409
- CN-Y- 2 840 933
- US-A1- 2007 255 281
- US-A1- 2007 255 281
- US-A1- 2007 270 876
- US-B1- 6 248 110

## Description

### Field of the Invention

The present invention relates to a kind of medical device, especially a filler for spine which is suit for comminuted fracture.

### Description of the Related Art

The widely-known technology is Chinese patent application No. 2008 20173700.0 (CN 201282981) patent from the present applicant in the existing technologies, the patent discloses a spreader and filler for surgery of compression fracture, this filler includes catheter and catheter holder for fastening catheter, there is a guide pin inside of the catheter, and the guide pin is fixed on the guide pin holder, a feed bag is set at one end of the caheter, the joint of the said feed bad connects to the catheter with thread, revolving the catheter must be done for the separation of feed bag and catheter of this kind of connection after surgery finished, that increases the difficulty of the surgery, in addition, the joint should be left in the body. Those are the shortcomings of the existing technologies.

### Summary of the invention

The purpose of the present invention is to offer a technical embodiment of a filler for spine aiming at the shortcomings of existing technologies. The feed bag is set at the outer wall of the catheter by outer thimble in the embodiments, which is convenient for surgery, when the surgery finished, the catheter and outer thimble could be drawn out and useless foreign matter need not be left in the body.

The embodiment is achieved through the following technical measures:
The filler for spine in the first embodiment includes a catheter and a catheter holder for fastening the catheter, there is a guide pin inside of the catheter, and the guide pin is fastened on the guide pin holder, a sealed or ventilate feed bag made of soft nonmetal materials according to biocompatibility or made of absorbing materials is set at the end of the catheter, the feature of the embodiment lies in that a outer thimble is set at the outer side of the catheter, the outer thimble is fixed on the outer thimble holder, a dismountable structure connects the outer thimble holder with the catheter holder, a reducing cone of tapered outer thimble at the open end of the said outer thimble connects to a little diameter outer thimble; the said catheter has a reducing cone corresponding to reducing cone of outer thimble at the reducing cone of outer thimble, and the reducing cone of catheter connects to a little diameter catheter; the said feed bag is set in the catheter through its open end, lies in between outer thimble and catheter and

The patent US 6,248,110 B1 discloses systems and methods, which treat fractured or diseased bone by deploying more than a single therapeutic tool into the bone. In one arrangement, the systems and methods deploy an expandable body in association with a bone cement nozzle into the bone, such that both occupy the bone interior at the same time. In another arrangement, the systems and methods deploy multiple expandable bodies, which occupy the bone interior volume simultaneously. Expansion of the bodies form cavity or cavities in cancellous bone in the interior bone volume.

The patent application US 2007/0255281 A1 discloses a method, which is provided for inserting an instrument set having a tapered configuration into a patient. Furthermore, a method provided for inserting a cannula having an external threaded portion into bone is disclosed. In yet another preferred aspect, a method is provided for forming and enlarging an opening in bone.

The patent application US 2007/0270876 A1 discloses a vertebra bone cement introduction system, which includes a main frame comprised of lateral handle and vertical insertion tube; a pouring frame comprised of lateral handle and vertical extension tube; an external injection device to inject cement into extension tube; a taper being meshed to extension tube; a polygonal hole disposed to taper and a recession in the mid of taper connected to a silicon gel bag containing cement; a locking rod penetrating the extension tube; and the lateral rod of the pouring frame being turned in reverse to disengage taper from extension tube while leaving taper and bag in a hole drilled on the bone.

fastened tightly at the outer wall of catheter by outer thimble. The specific feature of the embodiment also includes: the length of said little diameter catheter is 1-40mm, that of said little diameter outer thimble is 0-10mm, and that of said feed bag is between 10-50mm. The said feed bag is a monolayer, and a protective layer for feed bag is at the outer wall of the said feed bag, the dismountable connection between said outer thimble holder and catheter holder is achieved through a thread.

The filler for spine in the second example includes a catheter and a catheter holder for fastening the catheter, there is a guide pin inside of the catheter, and the guide pin is fastened on the guide pin holder, a sealed or ventilate feed bag made of soft nonmetal materials according to biocompatibility or made of absorbing materials is set at the end of the catheter, the feature of the embodiment lies in that a outer thimble is set at the outer side of the catheter, the outer thimble is fixed on the outer thimble holder, a dismountable structure connects the outer thimble holder with catheter holder an inside convex plate is set at the open end of outer thimble; said catheter has a reducing cone corresponding to inside convex plate of outer thimble at the inside convex plate of outer thimble, and the reducing cone of catheter connects to a little diameter catheter; said feed bag is set in the catheter through its open end, lies in between outer thimble and catheter and fastened tightly at the outer wall of catheter by outer thimble. Other specific features of the embodiment include: the length of the said little diameter catheter is 1-40mm, that of said little diameter outer thimble is 0.5-10mm, and that of said feed bag is between 10-50mm. The said feed bag is a monolayer, and a protective layer for feed bag is at the outer wall of the said feed bag, the dismountable connection between said outer thimble holder and catheter holder is achieved through a thread.

The beneficial effects of the fillers can be recognized by the above narration. In the two structures, the said feed bag is set at the outer wall of the catheter by outer thimble, so the bone materials such as ferroconcrete can be injected into the feed bag by catheter to support the spine of the patient. When the surgery finished, the catheter and outer thimble could be drawn out and useless foreign matter need not be left in the body. The difference of the two fillers only lies in the structure which presents the feed bag not to slip freely. In the first embodiment, the structure adopted is a reducing cone of a tapered outer thimble connects to a little diameter outer thimble at the open end of the outer thimble, and the catheter has a reducing cone corresponding to that of outer thimble at the reducing cone of outer thimble, and the reducing cone of catheter connects to a little diameter catheter. In the second example, the structure adopted is a inside convex plate at the open end of the outer thimble, and the catheter has a reducing cone corresponding to the inside convex plate at the inside convex plate of outer thimble, and reducing cone of catheter connects a little diameter catheter. The two embodiments adopt the changing diameter of feed bag when moving, to limit the feed bag not to move freely. Thus, the present invention has substantive features and progress compared with existing technologies, it is also obvious for its beneficial effects of implementation.

### Description of the drawings:

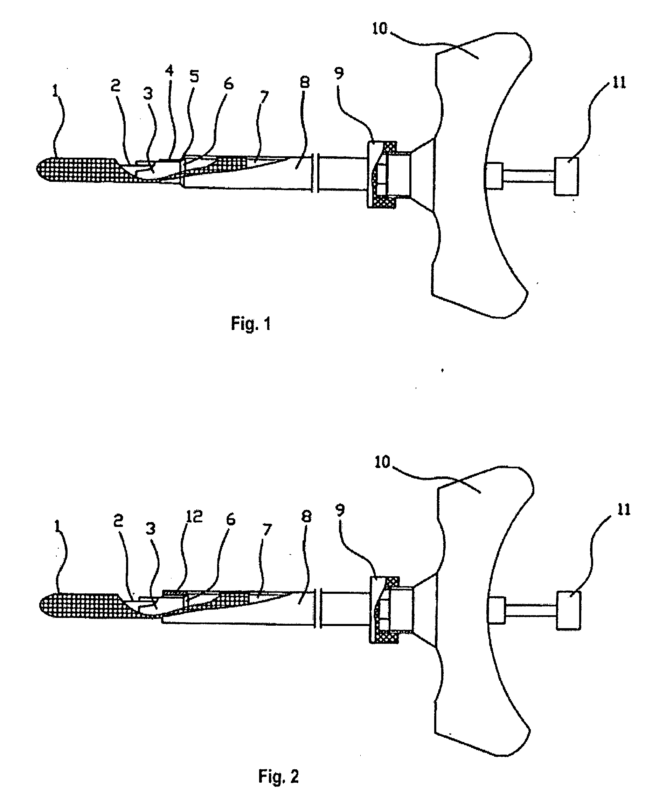

FIG. 1 is the partly sectional structure view of embodiment of the invention.

Where, 1 is feed bag, 2 is guide pin, 3 is little diameter catheter, 4 is little outer thimble, 5 is reducing cone of outer thimble, 6 is reducing cone of catheter, 7 is catheter, 8 is outer thimble, 9 is outer thimble holder, 10 is catheter holder, 11 is guide pin holder, 12 is inside convex plate of outer thimble.

### Detailed description of the invention

In order to clearly explain the technical features of the embodiment, the following will describe the embodiment by two embodiments and their drawings.

As it can be seen from the FIG. 1, the filler for spine of the embodiment includes a catheter 7 and a catheter holder 10 for fastening catheter 7, there is a guide pin 2 inside of the catheter 7, and the guide pin 2 is fastened on the guide pin holder 11, a sealed or ventilate feed bag 1 made of soft nonmetal materials according to biocompatibility or absorbing materials is set at the end of the catheter 2, a outer thimble 8 is set at the outer side of the said catheter 7 in this embodiment, and the outer thimble 8 is fixed on the outer thimble holder 9, a dismountable structure connects the outer thimble holder 9 and catheter holder 10, the thread connection is adopted in this embodiment, a reducing cone of tapered outer thimble 5 at the open end of the said outer thimble 8 connects to a little diameter outer thimble 4; the length of the little diameter outer thimble 4 is 0-10mm; said catheter 7 has a reducing cone of catheter 6 corresponding to reducing cone of outer thimble 5 at the reducing cone of outer thimble 5, and the reducing cone of catheter 6 connects to a little diameter catheter 3, the length of the little diameter catheter 3 is 1-40mm; In this embodiment, the length of the little diameter outer thimble 4 is 3mm, and that of the little diameter catheter 3 is 10mm. The said feed bag 1 is set in the catheter 7 through its open end, lies in between outer thimble 8 and catheter 7 and fastened tightly at the outer wall of catheter 7 by outer thimble 8. The length of the feed bag 1 is between 10-50mm. The said feed bag 1 can be a monolayer or a protective layer is added at the out wall of the feed bag1.

As it can be seen from FIG. 2, the filler for spine in the example includes a catheter 7 and a catheter holder 10 for fastening the catheter 7, there is a guide pin 2 inside of the catheter 7, and the guide pin 2 is fastened on the guide pin holder 11, a sealed or ventilate feed bag 1 made of soft nonmetal materials according to biocompatibility or absorbing materials is set at the end of the catheter 2, a outer thimble 8 is set at the outerside of the said catheter 7 in this embodiment, the outer thimble 8 is fixed on the outer thimble holder 9, a dismountable structure connects the outer thimble holder 9 and the catheter holder 8, the thread connection is adopted in this embodiment. A inside convex plate 12 is set at the open end of outer thimble 8, the length of the inside convex plate 12 is 0.5-10mm; the said catheter 7 has a reducing cone 6 corresponding to inside convex plate of outer thimble 12 at the inside convex plate of outer thimble 12, and the reducing cone 6 of catheter connects to a little diameter catheter 3; the length of the little catheter 3 is 1-40mm. In this embodiment, the length of the inside convex plate 12 of outer thimble is 5mm, and that of the little diameter of catheter 3 is 15mm. The said feed bag 1 is set in the catheter 7 through its open end, lies in between outer thimble 8 and catheter 7 and fastened tightly at the outer wall of catheter 7 by outer thimble 8, the length of feed bag 1 is between 10-50mem. The said feed bag 1 can be a monolayer or a protective layer is added at the out wall of the feed bag 1.

## Claims

1. A filler for spine includes a catheter (7) and a catheter holder (10) for fastening the catheter, wherein there is a guide pin (2) inside of the catheter, and the guide pin is fixed on the guide pin holder (11), a sealed or ventilate feed bag (1) made of soft nonmetal materials according to biocompatibility or absorbing materials is set at the end of the catheter (7), wherein a outer thimble (8) is set at the outer side of the said catheter, the outer thimble (8) is fixed on the outer thimble holder (9), a dismountable structure connects the outer thimble holder with catheter holder (10),
**characterized in that**
a reducing cone of tapered outer thimble (5) at the open end of the said outer thimble (8) connects to a little diameter outer thimble (4), said catheter has a reducing cone (6) corresponding to the reducing cone of outer thimble (5) at the reducing cone of outer thimble, and the reducing cone of catheter (6) connects to a little diameter catheter (3), said feed bag (1) is set in the catheter through its open end, lies in between outer thimble (8) and catheter (7) and fastened tightly at the outer wall of catheter by outer thimble.

2. The filler for spine of claim 1, wherein the length of the said little diameter catheter (3) is 1-40mm.

3. The filler for spine of claim 1 or 2, wherein the length of the said little diameter outer thimble (4) is 0-10mm.

4. The filler for spine of claim 1, wherein the length of the said feed bag (1) is 10-50mm.

5. The filler for spine of claim 1, wherein the said feed bag (1) is a monolayer.

6. The filler for spine of claim 1, wherein the said feed bag (1) has a protective layer at its outside wall.

7. The filler for spine of claim 1, wherein the dismountable connection between the said outer thimble holder (9) and the said catheter holder (10) is a thread one.

## Patentansprüche

1. Wirbelsäulenfüller, aufweisend einen Katheter (7) und einen Katheterhalter (10) zur Befestigung des Katheters, wobei es einen Führungsstift (2) innerhalb des Katheters gibt, und der Führungsstift am Führungsstiftshalter (11) befestigt ist, eine abgedichtete oder belüftete Zufuhrtasche (1) aus weichen Nichtmetall-Materialien entsprechend der Biokompatibilität oder saugfähigen Materialien am Ende des Katheters (7) angeordnet ist, wobei eine äußere Mantelhülse (8) an der Außenseite des Katheters angeordnet ist, die äußere Mantelhülse (8) am äußeren Mantelhülsenhalter (9) befestigt ist, eine abmontierbare Struktur den äußeren Mantelhülsenhalter mit dem Katheterhalter (10) verbindet,
**dadurch gekennzeichnet, dass**
ein sich verjüngender Kegel der spitz zulaufenden äußeren Mantelhülse (5) am offenen Ende der äußeren Mantelhülse (8) sich mit einer äußeren Mantelhülse (4) mit kleinem Durchmesser verbindet, der Katheter einen sich verjüngenden Kegel (6) aufweist, welcher dem sich verjüngenden Kegel der äußeren Mantelhülse (5) am sich verjüngenden Kegel der äußeren Mantelhülse entspricht, und der sich verjüngende Kegel des Katheters (6) mit einem Katheter (3) mit kleinem Durchmesser verbindet, die Zufuhrtasche (1) im Katheter durch dessen offenen Ende angeordnet ist, zwischen der äußeren Mantelhülse (8) und dem Katheter (7) gelegen und straff an der Außenwand des Katheters durch die äußere Mantelhülse befestigt ist.

2. Wirbelsäulenfüller nach Anspruch 1, wobei die Länge des Katheters (3) mit kleinem Durchmesser 1-40 mm beträgt.

3. Wirbelsäulenfüller nach Anspruch 1 oder 2, wobei die Länge der äußeren Mantelhülse (4) mit kleinem Durchmesser 0-10 mm beträgt.

4. Wirbelsäulenfüller nach Anspruch 1, wobei die Länge der Zufuhrtasche (1) 10-50 mm beträgt.

5. Wirbelsäulenfüller nach Anspruch 1, wobei die Zufuhrtasche (1) eine Monoschicht ist.

6. Wirbelsäulenfüller nach Anspruch 1, wobei die Zufuhrtasche (1) eine Schutzschicht an deren Außenwand aufweist.

7. Wirbelsäulenfüller nach Anspruch 1, wobei die abmontierbare Verbindung zwischen dem Halter der äußeren Zufuhrtasche (9) und dem Katheterhalter (10) eine Schraubverbindung ist.

## Revendications

1. Dispositif de remplissage de colonne vertébrale comportant un cathéter (7) et un support de cathéter (10) pour la fixation du cathéter, dans lequel il y a une broche de guidage (2) à l'intérieur dudit cathéter, et la broche de guidage est fixée sur le support de broche de guidage (11), un sachet d'alimentation (1) étanché ou ventilé fabriqué en matériaux non-métalliques conformément à leur biocompatibilité ou en matériaux absorbants est disposé à l'extrémité dudit cathéter (7), dans lequel une chaussette extérieure (8) est disposée sur le côté extérieur dudit cathéter, la chaussette extérieure (8) est fixée sur le support de chaussette extérieure (9), une structure démontable relie le support de chaussette extérieure au support de cathéter (10),
**caractérisé en ce que**
un cône réducteur de chaussette extérieure effilée (5) à l'extrémité ouverte de ladite chaussette extérieure (8) se raccorde à une chaussette extérieure de petit diamètre (4), ledit cathéter présente un cône réducteur (6) correspondant au cône réducteur de la chaussette extérieure (5) sur le cône réducteur de la chaussette extérieure, et le cône réducteur du cathéter (6) se raccorde à un cathéter de petit diamètre (3), ledit sachet d'alimentation (1) est disposé dans le cathéter à travers son extrémité ouverte, se situe entre la chaussette extérieure (8) et le cathéter (7) et est solidement fixé à la paroi extérieure du cathéter par la chaussette extérieure.

2. Dispositif de remplissage de colonne vertébrale selon la revendication 1, dans lequel la longueur dudit ledit cathéter (3) de petit diamètre est 1-40 mm.

3. Dispositif de remplissage de colonne vertébrale selon la revendication 1, dans lequel la longueur de ladite chaussette extérieure (4) de petit diamètre est 0-10 mm.

4. Dispositif de remplissage de colonne vertébrale selon la revendication 1, dans lequel la longueur dudit cathéter (1) de petit diamètre est 1-40 mm.

5. Dispositif de remplissage de colonne vertébrale selon la revendication 1, dans lequel ledit sachet d'alimentation (1) est une monocouche.

6. Dispositif de remplissage de colonne vertébrale selon la revendication 1, dans lequel ledit sachet d'alimentation (1) comporte une couche de protection sur sa paroi extérieure.

7. Dispositif de remplissage de colonne vertébrale selon la revendication 1, dans lequel le raccord démontable entre ledit support de chaussette extérieure (9) et ledit support de cathéter (10) est fileté.
